# FASCICULE DE BREVET EUROPEEN

(11) **EP 1 607 067 B1**
(45) Date de publication et mention de la délivrance du brevet: **04.01.2012**
(21) Numéro de dépôt: 05356103.1
(22) Date de dépôt: 14.06.2005
(51) Int. Cl.: A61F 2/40

(54) **Composant glénoïdien, jeu de tels composants et prothèse d'épaule incorporant un tel composant glénoïdien**
Schultergelenkpfannenteil, Satz von solchen Teilen und Schulterprothese mit einem solchen Schultergelenkpfannenteil
Glenoid component, set of such elements and shoulder prosthesis comprising such a glenoid component

(30) Priorité: 15.06.2004 FR 0406471
(43) Date de publication de la demande: 21.12.2005
(73) Titulaire: Tornier, 38330 Saint Ismier (FR)
(72) Inventeur: Tornier, Alain, 38330 Saint Isimier (FR); Sirveaux, François, 54600 Villers Les Nancy (FR); Walsch, Gilles, 69003 Lyon (FR); Mole, Daniel, 54000 Nancy (FR); Levigne, Christophe, 69300 Caluire (FR); Boileau, Pascal, 06200 Nice (FR); Favard, Luc, 37270 Montlouis (FR)
(74) Mandataire: Myon, Gérard Jean-Pierre

(56) Documents cités:
- EP-A- 0 549 480
- EP-A- 0 617 934
- EP-A- 1 064 890
- WO-A-91/07932
- CH-A- 507 704
- DE-A- 19 630 298
- FR-A- 2 726 994
- FR-A- 2 737 107
- FR-A- 2 835 425
- FR-A- 2 836 039
- US-A- 3 978 528
- US-A- 5 507 818
- US-A- 5 549 682

## Description

La présente invention a trait à un composant glénoïdien de prothèse d'épaule ainsi qu'à un jeu de tels composants pouvant être utilisés pour constituer une prothèse. L'invention a également trait à une prothèse d'épaule comprenant un tel composant.

Dans le domaine des prothèses d'épaule, il est connu, par exemple de US-A-3,978,528, de constituer une prothèse dite « inversée » dans laquelle une surface articulaire convexe solidaire de la glène et une surface articulaire concave solidaire de l'humérus coopèrent pour recréer une articulation au niveau de l'épaule. Dans ce genre de prothèses, le composant glénoïdien peut être formé, ainsi qu'il ressort de FR-A-2 835 425, d'une embase destinée à être immobilisée sur la glène et d'un élément destiné à être monté sur cette embase et définissant la surface d'articulation convexe.

Il est par ailleurs connu de FR-A-2 836 039 de prévoir une possibilité de montage d'un élément formant une surface d'articulation convexe sur un socle correspondant dans différentes positions, ce qui permet un réglage de la surface articulaire en hauteur par rapport à la glène.

L'embase ou le socle des composants glénoïdiens connus est pourvue d'une face dite « face arrière » destinée à venir en appui contre une surface réséquée de la glène qui est normalement sensiblement verticale lorsque le patient se tient debout. Or, il arrive que la partie supérieure de l'omoplate soit usée ou détruite, au point de modifier la cinématique de l'implant par le déplacement du centre de rotation d'origine, ce qui a pour conséquence de limiter les mouvements du bras du patient.

C'est à ces inconvénients qu'entend plus particulièrement remédier l'invention en proposant un composant glénoïdien qui permet d'assurer un bon d'articulation convexe, y compris lorsque la glène est endommagée ou usée en partie supérieure, voire en partie inférieure.

Dans cet esprit, l'invention concerne un composant glénoïdien de prothèse d'épaule qui forme une surface d'articulation convexe centrée sur un axe de symétrie, ce composant comprenant une embase, apte à être immobilisée sur la glène d'une épaule alors que l'axe de symétrie de la surface d'articulation convexe est non perpendiculaire à une face arrière de l'embase qui est destinée à venir en appui contre la glène. Ce composant est caractérisé en ce que la surface d'articulation convexe est définie par un élément prévu pour être monté sur cette embase, en ce que le composant comprend un doigt de centrage de cet élément définissant la surface d'articulation convexe et en ce que l'embase est percée d'un logement de réception du doigt de centrage.

Grâce à l'invention, la surface d'articulation convexe peut être inclinée vers le bas ou « déversée » par rapport à la face arrière du composant, ce qui permet d'orienter de façon correcte cette surface articulaire, y compris lorsque la surface d'appui créée dans la glène par résection n'est pas parallèle à la direction de la colonne vertébrale du patient. L'invention permet donc de « rattraper » ou de « compenser » un défaut de parallélisme entre la surface réséquée de la glène et l'axe de la colonne vertébrale du patient.

Selon des aspects avantageux mais non obligatoires, un composant glénoïdien peut incorporer une ou plusieurs des caractéristiques suivantes prises dans toute combinaison techniquement admissible :
- En configuration assemblée du composant et lorsque la face arrière est verticale, l'axe de symétrie de la surface d'articulation convexe est dirigé vers le bas en s'éloignant de la face arrière.
- L'embase est pourvue d'une face avant sensiblement plane dans laquelle est percé un logement de réception d'un doigt de centrage de l'élément formant la surface d'articulation convexe, ce logement étant centré sur un axe sensiblement perpendiculaire à cette face avant, cette face avant n'étant pas parallèle à la face arrière de l'embase. Dans ce cas, les faces avant et arrière de l'embase peuvent former entre elles un angle compris entre 2° et 18°. L'embase peut être pourvue d'une surface axisymétrique centrée sur un axe perpendiculaire à sa face avant, cette surface étant apte à coopérer avec une surface interne de l'élément précité pour son centrage et son immobilisation sur l'embase.
- Le composant peut être pourvu d'une queue d'ancrage qui s'étend selon une direction qui n'est pas perpendiculaire à une partie au moins de sa face arrière.
- L'élément qui forme la surface d'articulation convexe est pourvu d'une jupe non symétrique par rapport à l'axe de la surface précitée, qui prolonge cette surface et dans laquelle est défini, au moins en partie, un logement de la réception d'une partie au moins de l'embase. Cette jupe peut être sensiblement en forme de tronçon de tore. L'élément qui définit la surface d'articulation convexe est avantageusement pourvu d'un perçage de passage d'un organe de manoeuvre d'un moyen de solidarisation de cet élément sur l'embase, ce passage s'étendant sensiblement selon une direction globalement perpendiculaire à cette surface mais non confondue avec son axe de symétrie.

L'invention concerne également un jeu de composants glénoïdiens pour prothèse d'épaule du type défini ci-dessus qui permet à un chirurgien de sélectionner un composant de géométrie adaptée en fonction de la configuration effective de la glène une fois celle-ci réséquée. Ce jeu de composants est caractérisé en ce que l'orientation de l'axe de symétrie de la surface d'articulation convexe, par rapport à une face arrière de chaque composant destinée à venir en appui contre la glène, est variable d'un composant à l'autre.

Selon une première variante de réalisation, les faces avant et arrière des embases des composants peuvent ne pas être parallèles entre elles, auquel cas l'angle entre ces faces avant et arrière est différent d'un composant à l'autre.

Selon une autre variante, les éléments de ces composants qui forment une surface articulaire convexe peuvent être chacun pourvus d'une jupe, comme mentionné ci-dessus, les dimensions des jupes étant différentes d'un composant à l'autre.

L'invention concerne également une prothèse totale d'épaule qui comprend un composant glénoïdien tel que décrit ci-dessus.

La description concerne enfin une méthode de pose d'un composant glénoïdien de prothèse totale d'épaule, un tel composant définissant une surface articulaire convexe centrée sur un axe de symétrie, alors que cette méthode comprend des étapes consistant à :
- fraiser la glène du patient pour créer une surface d'appui du composant ;
- sélectionner, parmi plusieurs composants glénoïdiens dont les axes de symétrie des surfaces articulaires convexes sont orientées différemment par rapport à leur face d'appui contre la surface réséquée de la glène, un composant qui peut être plaqué contre cette surface dans une position telle que l'axe de symétrie précité est globalement perpendiculaire à la direction de la colonne vertébrale du patient et
- immobiliser le composant glénoïdien sélectionné sur la glène dans la position précitée.

L'invention sera mieux comprise et d'autres avantages de celle-ci apparaîtront plus clairement à la lumière de la description qui va suivre de cinq modes de réalisation d'un composant glénoïdien et de deux modes de réalisation d'un jeu de composants glénoïdiens conformes à son principe, donnée uniquement à titre d'exemple et faite en référence aux dessins annexés dans lesquels :
- la figure 1 est une représentation schématique de principe d'une prothèse d'épaule conforme à l'invention implantée sur un patient dans une première configuration ;
- la figure 2 est une vue analogue à la figure 1 pour une prothèse également conforme à l'invention implantée dans une seconde configuration ;
- la figure 3 est une vue de côté éclatée du composant glénoïdien utilisé dans la prothèse de la figure 2 ;
- la figure 4 est une vue en perspective d'une embase appartenant au composant représenté à la figure 3 ;
- la figure 5 est une représentation schématique de principe d'un jeu de composants glénoïdiens incorporant celui représenté aux figures 3 et 4 ;
- la figure 6 est une vue analogue à la figure 3 pour un composant conforme à un second mode de réalisation de l'invention ;
- la figure 7 est une vue analogue à la figure 3 pour un composant conforme à un troisième mode de réalisation de l'invention ;
- la figure 8 représente un composant glénoïdien conforme à un quatrième mode de réalisation de l'invention dont l'embase est représentée en vue de côté et dont l'élément définissant la surface articulaire convexe est représenté en coupe transversale ;
- la figure 9 est une vue en perspective éclatée du composant de la figure 8 ;
- la figure 10 est une vue analogue à la figure 8 pour un composant conforme à un cinquième mode de réalisation de l'invention ; et
- la figure 11 est une vue analogue à la figure 9 pour le composant de la figure 10.

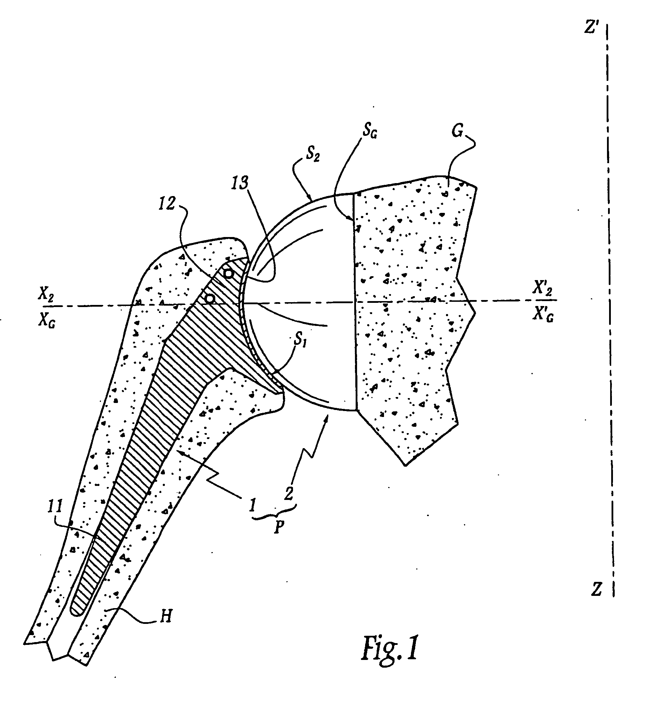

La prothèse P représentée à la figure 1 comprend un composant huméral 1 comportant une tige 11 destinée à être ancrée dans le canal médullaire de l'humérus H ainsi qu'une partie métaphysaire 12 dans laquelle est immobilisée une coupelle 13 en polyéthylène définissant une surface articulaire concave S₁ sensiblement en forme de tronçon de sphère.

Selon une variante non représentée de l'invention, le composant 1 peut être dépourvu de coupelle 13, la surface S₁ étant formée par la partie métaphysaire qui est métallique.

La prothèse P comprend également un composant glénoïdien 2 qui définit une surface articulaire convexe S₂ sensiblement en forme de demi sphère.

Pour la clarté du dessin, le composant 1 est représenté en coupe, alors que le composant 2 est représenté en vue extérieure sur la figure 1.

La surface S₂ est sensiblement en forme de demi sphère et l'on note X₂-X'₂ l'axe de symétrie sur lequel est centrée cette surface.

On note par ailleurs Z-Z' un axe vertical passant par le centre de la colonne vertébrale du patient qui se tient debout.

On note S_{G} la surface fraisée de la glène G contre laquelle vient en appui le composant 2 lorsqu'il est monté sur la glène.

Normalement, la surface S_{G} est sensiblement parallèle à l'axe Z-Z', de telle sorte qu'un axe X_{G}-X'_{G} normal à la surface S_{G} et passant par son centre est sensiblement perpendiculaire à l'axe Z-Z', c'est-à-dire sensiblement horizontal lorsque le patient se tient debout. Dans la configuration de la figure 1 qui correspond à une configuration nominale d'implantation, les axes X_{G}-X'_{G} et X₂-X'₂ sont confondus et tous deux horizontaux.

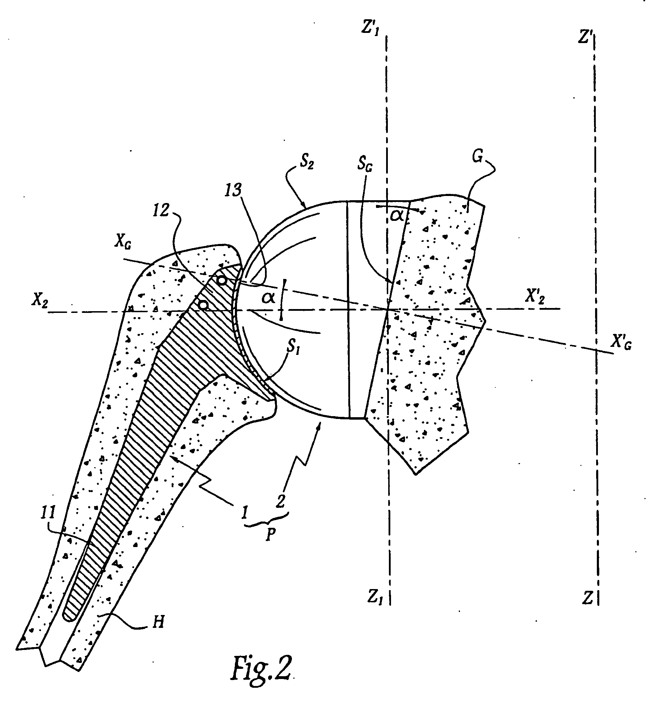

Cependant, comme représenté à la figure 2, il arrive que la surface S_{G} ne soit pas réellement parallèle à l'axe Z-Z', notamment du fait d'une usure de la glène G ou d'une destruction partielle de celle-ci en partie haute, voire en partie basse. Dans ce cas, la surface S_{G} forme un angle α non nul par rapport à une droite Z₁-Z'₁ parallèle à l'axe Z-Z' et passant par le centre M_{G} de la surface S_{G}. Lorsque le patient se tient debout, l'axe X_{G}-X'_{G} forme, par rapport à l'horizontale, le même angle α.

Conformément à l'invention, le composant 2 est configuré pour que l'axe X₂-X'₂ de la surface S₂ soit sensiblement horizontal lorsque le patient se tient debout, malgré l'orientation non optimale de la surface S_{G}.

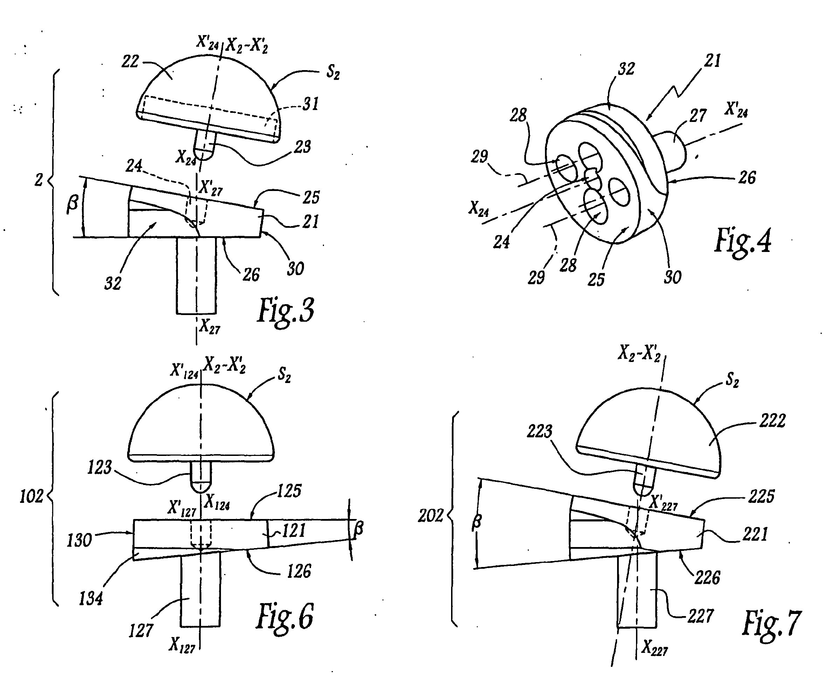

Le composant 2 est représenté en vue de côté éclatée à la figure 3 et comprend une embase ou platine 21 destinée à être fixée sur la glène G, ainsi qu'un élément métallique 22, parfois dénommé « tête », qui définit la surface S₂ et qui est pourvu d'un doigt de centrage 23 destiné à pénétrer dans un logement 24 ménagé dans l'embase 21 et centré sur un axe X₂₄-X'₂₄ perpendiculaire à la face avant 25 de l'embase 21 qui est sensiblement plane et orientée à l'opposé de la glène lorsque l'embase est montée sur la glène.

On note 26 la face arrière de l'embase 21 qui porte contre la surface S_{G} en configuration montée de l'embase.

Une queue d'ancrage 27 s'étend à partir de la surface 26 selon une direction parallèle à un axe X₂₇-X'₂₇ perpendiculaire à la surface 26.

L'embase 21 est également percée de quatre orifices 28 de passage de quatre vis 29 représentées uniquement par leurs traits d'axe à la figure 4.

La face avant 25 est en forme de disque centré sur l'axe X₂₄-X'₂₄ et bordée par une surface 30 tronconique, centrée sur l'axe X₂₄-X'₂₄ et convergente à l'opposé de la queue 27.

La surface 30 s'étend tout autour de la face avant 25 mais rejoint la face arrière 26 sur une partie seulement de la périphérie de l'embase 21.

La face 26 est sensiblement plane et les faces 25 et 26 ne sont pas parallèles entre elles. On note β l'angle non nul formé entre les faces 25 et 26.

Le caractère incliné de la face avant 25 par rapport à la face arrière 26 de l'embase 21 permet de « rattraper » complètement ou partiellement le caractère incliné par rapport à la droite Z₁-Z'₁ de la surface S_{G}, pour autant que la partie la moins épaisse de l'embase 21, qui est représentée sur la droite de la figure 3, est disposée au voisinage de la partie inférieure de la surface S_{G}, c'est-à-dire de la partie située du côté des côtes du patient.

Lorsque l'embase ou platine 21 a été ancrée sur la glène G comme indiqué ci-dessus grâce à la queue 27 et à la mise en place des quatre vis 29, l'élément 22 peut être mis en place en introduisant le doigt 23 dans le logement 24 et en faisant porter une surface interne 31 de l'élément 22 représenté en pointillés uniquement à la figure 3 contre la surface 30. Les géométries des surfaces 30 et 31 sont adaptées pour obtenir un verrouillage à la façon d'un cône Morse.

Ainsi, l'orientation relative des faces 25 et 26 permet d'orienter l'axe X₂-X'₂ de la surface S₂ vers le bas à la figure 2 par rapport à l'axe X_{G}-X'_{G} dans sa partie qui dépasse de la glène, c'est-à-dire de ramener cet axe en configuration sensiblement horizontale, alors que la surface S_{G} n'est pas parallèle à l'axe Z-Z'.

Compte tenu de l'orientation relative des faces 25 et 26, la surface 30 ne borde la face 25 que sur une fraction de la hauteur de la partie de l'embase située entre les faces 25 et 26. On note 32 la portion de surface périphérique de l'embase 21 qui n'est pas formée par la partie 30. Cette surface est gauche.

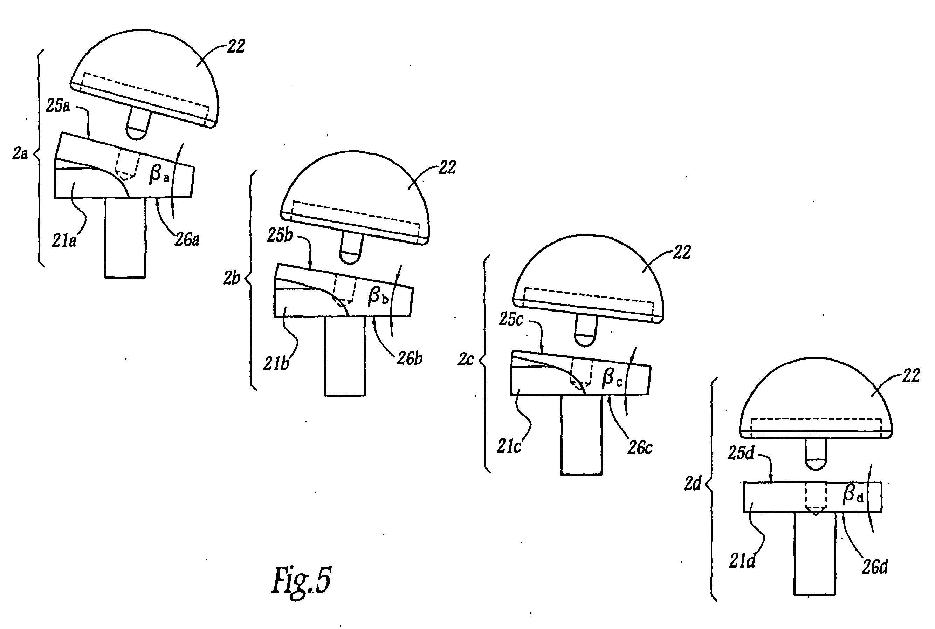

Comme il ressort plus particulièrement de la figure 5, un jeu de composants glénoïdiens conforme à l'invention peut incorporer plusieurs composants glénoïdiens 2a, 2b, 2c et 2d dont les embases 21a, 21b, 21c et 21d ont des faces avant 25a, 25b, 25c et 25d qui font des angles βa, βb, βc, βd différents par rapport à leurs faces arrières respectives 26a, 26b, 26c et 26d. On note que les faces avant et arrière de l'embase 21d sont sensiblement parallèles, l'angle βd entre celles-ci est alors nul.

L'élément 22 associé à chaque embase 21a, 21b, 21c et 21d peut être le même ou être différent d'un composant à l'autre.

Ainsi, lorsqu'un chirurgien pose une prothèse d'épaule P, il peut, en fonction de l'orientation relative de la surface S_{G} et de l'axe Z-Z', choisir un composant glénoïdien dont l'embase comporte des faces avant et arrière orientées d'une façon permettant de rattraper l'essentiel du défaut d'orientation de la surface S_{G}.

Bien entendu, le nombre de composants glénoïdiens d'un jeu tel que celui représenté à la figure 5 n'est pas nécessairement de quatre. Il peut être choisi en fonction de la précision recherchée. En outre, il n'est pas obligatoire que, dans un tel jeu, une platine ait des surfaces parallèles entre elles, comme celle représentée sur la droite de la figure 5. On note cependant que le composant glénoïdien représenté sur la droite de la figure 5 peut être utilisé lorsque la prothèse P doit être implantée dans la configuration de la figure 1.

La pose d'une prothèse totale d'épaule est facilitée par l'utilisation d'un tel jeu de composants dans la mesure où le chirurgien peut sélectionner un composant glénoïdien effectivement adapté à la morphologie du patient puis immobiliser ce composant dans une position telle que l'axe de symétrie de la surface articulaire convexe est sensiblement perpendiculaire à l'axe longitudinal de la colonne vertébrale du patient.

Dans le second mode de réalisation de l'invention représenté à la figure 6, les éléments analogues à ceux du premier mode de réalisation portent des références identiques augmentées de 100. Le composant glénoïdien 102 de ce mode de réalisation comprend un élément 122 qui porte une surface articulaire convexe sensiblement hémisphérique, cet élément étant identique à celui du premier mode de réalisation. L'embase 121 du composant 102 comprend également une queue d'ancrage 127. Cette queue d'ancrage est centrée sur un axe X₂₇-X'₂₇ perpendiculaire à la face avant 125 de l'embase 121 qui est plane et bordée par une surface tronconique 130. L'embase 121 est pourvue d'une logement 124 de réception d'un doigt 123 appartenant à l'élément 122, ce doigt étant centré sur l'axe X₂-X'₂ de symétrie de la surface S₂. L'axe central X₁₂₄-X'₁₂₄ du logement 124 est confondu avec l'axe central X₁₂₇-X'₁₂₇ de la queue 127.

Ce mode de réalisation diffère du précédent en ce que la face arrière 126 de l'embase 121 n'est pas perpendiculaire à l'axe X₁₂₇-X'₁₂₇, de telle sorte qu'un angle β non nul existe entre les faces 125 et 126.

Dans ce mode de réalisation, on peut considérer que la face arrière 126 est pourvue d'un « talon » ou coin 134 qui sert à compenser l'orientation non optimale de la surface S_{G}.

Dans le troisième mode de réalisation de l'invention représenté à la figure 7, les éléments analogues à ceux du premier mode de réalisation portent des références identiques augmentées de 200. Le composant glénoïdien 202 de ce mode de réalisation comprend une embase 221 ainsi qu'un élément 222 qui forme une surface articulaire S₂ sensiblement hémisphérique et centrée sur un axe X₂-X'₂ sur lequel est également centré un doigt de centrage 223.

Les faces avant 225 et arrière 226 de l'embase 221 ne sont pas parallèles entre elles et définissent un angle β non nul. Ce mode de réalisation incorpore certains éléments des premier et second modes de réalisation à savoir que les surfaces 225 et 226 sont toutes deux inclinées, dans des sens différents, par rapport à un axe longitudinal X₂₂₇-X'₂₂₇ d'une queue d'ancrage 227 de l'embase 221 dans la glène.

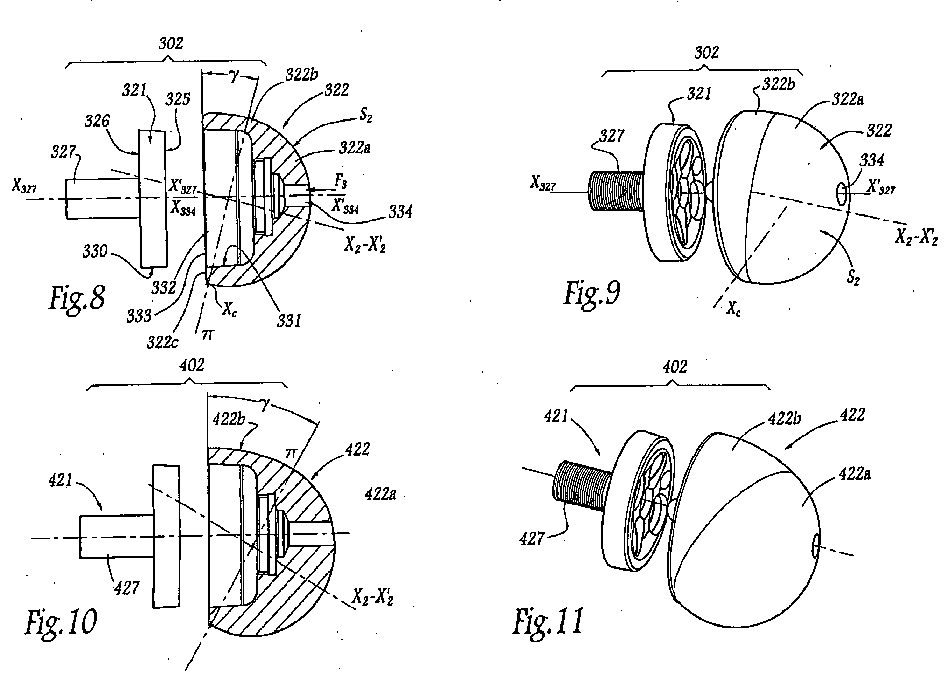

Dans le quatrième mode de réalisation de l'invention représenté aux figures 8 et 9, les éléments analogues à ceux du premier mode de réalisation portent des références identiques augmentées de 300. Le composant glénoïdien 302 de ce mode de réalisation comprend une embase 321 analogue à celle du composant représenté sur la droite de la figure 5, c'est-à-dire dont les faces avant 325 et arrière 326 sont sensiblement parallèles entre elles et perpendiculaires à un axe central X₃₂₇-X'₃₂₇ d'une queue d'ancrage 327.

Un élément 322 destiné à être monté sur l'embase 321 définit une surface S₂ sensiblement en forme de demi sphère et centrée sur un axe X₂-X'₂ qui n'est pas parallèle à l'axe X₃₂₇-X'₃₂₇ en configuration montée de l'élément 322 sur l'embase 321. Pour ce faire, la portion 322a de l'élément 322 qui définit la surface S₂ est prolongée par une jupe 322b en forme de tronçon de tore centré sur un axe X_{c} perpendiculaire à l'axe X₂-X'₂ et tangent à la surface articulaire convexe S₂ au voisinage d'une zone d'intersection entre cette surface S₂ et un plan II perpendiculaire à l'axe X₂-X'₂ et passant par le centre C₂ de la surface S₂. On note γ l'amplitude angulaire de la jupe 322, c'est-à-dire l'angle entre le plan n et la face arrière 322c de l'élément 322 destinée à être tournée vers la surface réséquée de la glène en configuration montée du composant 302.

Un logement 332 est ménagé à l'intérieur de l'élément 322, à la fois dans la jupe 322b et dans la portion 322a. Ce logement est destiné à recevoir la partie de l'embase 321 définie entre les surfaces 325 et 326. Le logement 332 est bordé par une surface 331 tronconique et convergente en direction de la surface S₂, alors qu'une surface 330 de même géométrie est prévue sur l'élément 321 entre les faces 325 et 326.

On note 333 l'ouverture d'entrée circulaire du logement 332.

La surface S₂ est percée d'un passage 334 permettant l'introduction d'un outil dans le sens de la flèche F₃ jusqu'à l'intérieur de l'élément 322, ce qui permet de manoeuvrer une vis non représentée d'immobilisation de l'élément 322 sur l'embase 321. Une telle vis peut, notamment, commander le déplacement d'un doigt tel que le doigt 23 du premier mode de réalisation, qui est alors fileté, pour l'amener en prise avec une partie taraudée de l'embase 321, ceci conformément à l'enseignement technique de FR-A-2 835 425.

On note X₃₃₄-X'₃₃₄ l'axe longitudinal du passage 334 . Cet axe est perpendiculaire à la surface S₂ et décalé de l'angle γ par rapport à l'axe X₂-X'₂.

Dans le cinquième mode de réalisation de l'invention représenté à la figure 10, les éléments analogues à ceux du premier mode de réalisation portent des références identiques augmentées de 400. Le composant glénoïdien 402 de ce mode de réalisation est réalisé selon le même principe que celui du quatrième mode de réalisation avec une embase 421 identique à l'embase 321 et un élément 422 qui définit une surface S₂ centrée sur un axe X₂-X'₂ incliné vers le bas à la figure 10. Ce mode de réalisation diffère du précédent en ce que l'amplitude angulaire γ de la jupe 422b, qui prolonge la portion 422a de l'élément 422 définissant la surface S₂, est plus important que dans le mode de réalisation des figures 8 et 9, ce qui permet d'augmenter l'effet de déversement de la surface S₂ par rapport à la face arrière 426 de l'embase 421.

Les composants représentés aux figures 8 à 11 peuvent être considérés comme appartenant à un même jeu de composants glénoïdiens permettant au chirurgien de sélectionner le composant le plus adapté en fonction de la valeur de l'angle γ et de l'orientation de la surface réséquée S_{G} de la glène par rapport à l'axe longitudinal Z-Z' de la colonne vertébrale du patient.

Bien entendu, le nombre de composants d'un tel jeu n'est pas limité à deux.

Les caractéristiques des différents modes de réalisation représentés peuvent être combinées entre elles en ce sens qu'une embase ou platine à faces avant et arrière non parallèles pourrait être utilisée avec un élément pourvu d'une jupe prolongeant la portion de cet élément définissant une surface articulaire convexe axisymétrique.

L'invention permet également une implantation correcte d'un composant glénoïdien lorsque la partie inférieure de l'omoplate est endommagée. Il suffit alors de retourner le composant par rapport à la configuration représentée à la figure 2.

L'invention a été représentée avec des composants glénoïdiens bi-partites.

## Revendications

1. Composant glénoïdien (2 ; 102 ; 202 ; 302 ; 402) de prothèse d'épaule formant une surface d'articulation convexe (S₂) centrée sur un axe de symétrie (X₂-X'₂), ce composant comprenant une embase (21 ; 121 ; 221 ; 321 ; 421), apte à être immobilisée sur la glène d'une épaule, alors que ledit axe de symétrie (X₂-X'₂) est non perpendiculaire (β,γ) à une face arrière (26 ; 126 ; 226 ; 326 ; 426) de ladite embase (21 ; 121 ; 221 ; 321) destinée à venir en appui contre la glène (G), **caractérisé en ce que** la surface d'articulation convexe (S₂) est définie par un élément (22 ; 122 ; 222 ; 322 ; 422) prévu pour être monté sur ladite embase , **en ce que** le composant comprend un doigt (23) de centrage de l'élément définissant ladite surface d'articulation convexe (S₂) et **en ce que** l'embase est percée d'un logement (24) de réception du doigt de centrage (23).

2. Composant selon la revendication 1, **caractérisé en ce qu'**en configuration montée dudit composant (2 ; 102 ; .202 ; 302 ; 402) et lorsque ladite face arrière (26 ; 126 ; 226 ; 326 ; 426) est verticale, ledit axe (X₂-X'₂) est dirigé vers le bas en s'éloignant de ladite face arrière.

3. Composant selon l'une des revendications précédentes, **caractérisé en ce que** ladite embase (21 ; 121 ; 221) est pourvue d'une face avant (25 ; 125 ; 225) sensiblement plane dans laquelle est percé un logement (24 ; 124) de réception d'un doigt (23 ; 123 ; 223) de centrage dudit élément (22 ; 122 ; 222) formant la surface d'articulation convexe (S₂), ledit logement étant centré sur un axe (X₂₄-X'₂₄; X₁₂₄-X'₁₂₄) sensiblement perpendiculaire à ladite face avant, ladite face avant n'étant pas parallèle (β) à la face arrière (26 ; 126 ; 226) de ladite embase.

4. Composant selon la revendication 3, **caractérisé en ce que** lesdites faces avant (25 ; 125 ; 225) et arrière (26 ; 126 ; 226) forment ensemble un angle (β) compris entre 2° et 18°.

5. Composant selon l'une des revendications 3 ou 4, **caractérisé en ce que** ladite embase (21 ; 121 ; 221) est pourvue d'une surface axisymétrique (30 ; 130) centrée sur un axe (X₂₄-X'₂₄ ; X₁₂₄-X'₁₂₄) perpendiculaire à ladite face avant (25 ; 125 ; 225), ladite surface étant apte à coopérer avec une surface interne (31) dudit élément (22 ; 122 ; 222) pour le centrage et l'immobilisation dudit élément sur ladite embase.

6. Composant selon l'une des revendications précédentes, **caractérisé en ce qu'**il est pourvu d'une queue d'ancrage (127 ; 227) qui s'étend selon une direction qui n'est pas perpendiculaire à une partie au moins de ladite face arrière (126 ; 226).

7. Composant selon l'une des revendications précédentes, **caractérisé en ce que** ledit élément (322 ; 422) définissant la surface d'articulation convexe (S₂) est pourvu d'une jupe (322b ; 422b) non symétrique (γ) par rapport audit axe, qui prolonge ladite surface articulaire convexe (S₂) et dans laquelle est défini, au moins en partie, un logement (332) de réception d'une partie au moins de ladite embase (321 ; 421).

8. Composant selon la revendication 7, **caractérisé en ce que** ladite jupe (322b ; 422b) est sensiblement en forme de tronçon de tore.

9. Composant selon l'une des revendications 7 ou 8, **caractérisé en ce que** ledit élément (322 ; 422) est pourvu d'un perçage (334) de passage (F₃) d'un organe de manoeuvre d'un moyen de solidarisation dudit élément sur ladite embase (321 ; 421), ledit perçage s'étendant sensiblement selon une direction (X₃₃₄-X'₃₃₄) globalement perpendiculaire à ladite surface (S₂) et non confondue (γ) avec ledit axe (X₂-X'₂).

10. Jeu de composants glénoïdiens selon la revendication 1 pour prothèse d'épaule dans lequel chaque composant comprend une embase (21 ; 121 ; 221 ; 321 ; 421), apte à être immobilisée sur la glène d'une épaule, et un élément (22 ; 122 ; 222 ; 322 ; 422) prévu pour être monté sur ladite embase et formant une surface d'articulation convexe centrée sur un axe de symétrie (X₂-X'₂), **caractérisé en ce que** l'orientation (β,γ) dudit axe de symétrie (X₂-X'2), par rapport à la face arrière (26 ; 126 ; 226 ; 326 ; 426) de chaque composant destinée à venir en appui contre la glène (G), est variable d'un composant à l'autre (2a-2d ; 302, 402).

11. Jeu de composants selon la revendication 10, **caractérisé en ce que** les composants sont selon la revendication 3 et **en ce que** l'angle (β) entre les faces avant (25 ; 125 ; 225) et arrière (26 ; 126 ; 226) des embases (21 ; 121 ; 221) est différent d'un composant à l'autre (2a-2d).

12. Jeu de composants selon la revendication 10, **caractérisé en ce que** les composants sont selon la revendication 7 et **en ce que** les dimensions (γ) des jupes (322b, 422b) sont différentes d'un composant à l'autre (302, 402).

13. Prothèse totale d'épaule, **caractérisée en ce qu'**elle comprend un composant glénoïdien (2 ; 102 ; 202 ; 302 ; 402) selon l'une des revendications 1 à 9.

## Claims

1. A shoulder prosthesis glenoid component (2; 102; 202; 302; 402) forming a convex joint surface (S₂) centred on an axis of symmetry (X₂-X'₂), this component comprising a base (21; 121; 221; 321; 421), suited to being immobilised on the glenoid of a shoulder, while said axis of symmetry (X₂-X'₂) is not perpendicular (β, γ) to a rear face (26; 126; 226; 326; 426) of said base (21; 121; 221; 321) designed to come to bear against the glenoid (G), **characterised in that** the convex joint surface (S₂) is defined by an element (22; 122; 222; 322; 422) intended to be mounted on said base, **in that** the component comprises a finger (23) for centring the element defining said convex joint surface (S₂) and **in that** a receptacle (24) is formed in the base for receiving the centring finger (23).

2. A component according to claim 1, **characterised in that** when said component (2; 102; 202; 302; 402) is in the mounted configuration and when said rear face (26; 126; 226; 326; 426) is vertical, said axis (X₂-X'₂) is directed downwards away from said rear face.

3. A component according to any one of the preceding claims, **characterised in that** said base (21; 121; 221) is provided with a substantially planar front face (25; 125; 225), in which a receptacle (24; 124) is formed for receiving a finger (23; 123; 223) for centring said element (22; 122; 222) forming the convex joint surface (S₂), said receptacle being centred on an axis (X₂₄-X'₂₄; X₁₂₄-X'₁₂₄) substantially perpendicular to said front face, said front face not being parallel (β) to the rear face (26; 126; 226) of said base.

4. A component according to claim 3, **characterised in that** said front face (25; 125; 225) and rear face (26; 126; 226) together form an angle (β) of between 2° and 18°.

5. A component according to either one of claims 3 or 4, **characterised in that** said base (21; 121; 221) is provided with an asymmetric surface (30; 130) centred on an axis (X₂₄-X'₂₄; X₁₂₄-X'₁₂₄) perpendicular to said front face (25; 125; 225), said surface being suited to cooperating with an internal surface (31) of said element (22; 122; 222) for centring and immobilising said element on said base.

6. A component according to any one of the preceding claims, **characterised in that** an anchoring stem (127; 227) is provided, which extends in a direction which is not perpendicular to part at least of said rear face (126; 226).

7. A component according to any one of the preceding claims, **characterised in that** said element (322; 422) defining the convex joint surface (S₂) is provided with a skirt (322b; 422b) which is not symmetrical (γ) relative to said axis, which extends said convex joint surface (S2) and in which there is defined, at least in part, a receptacle (332) for receiving part at least of said base (321; 421).

8. A component according to claim 7, **characterised in that** said skirt (322b; 422b) substantially takes the form of a section of a torus.

9. A component according to either one of claims 7 or 8, **characterised in that** said element (322; 422) is provided with a hole (334) for passage (F₃) of a member for actuating a means of firmly attaching said element to said base (321; 421), said hole extending substantially in a direction (X₃₃₄-X'₃₃₄) which is overall perpendicular to said surface (S2) and not merged (γ) with said axis (X₂-X'₂).

10. A set of glenoid components according to claim 1 for a shoulder prosthesis in which each component comprises a base (21; 121; 221; 321; 421) suited to being immobilised on the glenoid of a shoulder, and an element (22; 122; 222; 322; 422) intended to be mounted on said base and forming a convex joint surface centred on an axis of symmetry (X₂-X'₂), **characterised in that** the orientation (β, γ) of said axis of symmetry (X₂-X'₂), relative to the rear face (26; 126; 226; 326; 426) of each component intended to come to bear against the glenoid (G), is variable from one component to another (2a-2d; 302, 402).

11. A set of components according to claim 10, **characterised in that** the components are as claimed in claim 3 and **in that** the angle (β) between the front faces (25; 125; 225) and rear faces (26; 126; 226) of the bases (21; 121; 221) is different from one component to another (2a-2d).

12. A set of components according to claim 10, **characterised in that** the components are as claimed in claim 7 and **in that** the dimensions (γ) of the skirts (322b, 422b) are different from one component to another (302, 402).

13. A total shoulder prosthesis, **characterised in that** it comprises a glenoid component (2; 102; 202; 302; 402) according to any one of claims 1 to 9.

## Patentansprüche

1. Gelenkpfannenkomponente (2; 102; 202; 302; 402) für Schulterprothese, die eine konvexe Gelenkoberfläche (S₂) bildet, die auf eine Symmetrieachse (X₂-X'₂) zentriert ist, wobei diese Komponente eine Basis (21; 121; 221; 321; 421) aufweist, die dazu geeignet ist, an einer Gelenkpfanne einer Schulter immobilisiert zu werden, während die besagte Symmetrieachse (X₂-X'₂) zu einer hinteren Fläche (26; 126; 226; 326; 426) der besagten Basis (21; 121; 221; 321), die dazu bestimmt ist, sich an der Gelenkpfanne (G) abzustützen, nicht senkrecht (β, γ) ist, **dadurch gekennzeichnet, dass** die konvexe Gelenkoberfläche (S₂) durch ein Element (22; 122; 222; 322; 422) definiert ist, das dazu vorgesehen ist, an der besagten Basis montiert zu werden, dass die Komponente einen Zentrierzapfen (23) zum Zentrieren des die besagte konvexe Gelenkoberfläche (S₂) definierenden Elements aufweist und dass die Basis von einem Aufnahmesitz (24) für die Aufnahme des Zentrierungszapfens (23) durchsetzt ist.

2. Komponente nach Anspruch 1, **dadurch gekennzeichnet, dass** in der montierten Konfiguration der besagten Komponente (2; 102; 202; 302; 402) und dann, wenn die besagte hintere Fläche (26; 126; 226; 326; 426) vertikal ist, die besagte Achse (X₂-X'₂) von der besagten hinteren Fläche wegweisend nach unten gerichtet ist.

3. Komponente nach einem der vorhergehenden Ansprüche, **dadurch gekennzeichnet, dass** die besagte Basis (21; 121; 221) mit einer vorderen Fläche (25; 125; 2,25) versehen ist, die im Wesentlichen eben ist und in der ein Sitz (24; 124) für die Aufnahme eines Zapfens (23; 123; 223) für die Zentrierung des besagten, die konvexe Gelenkoberfläche (S₂) bildenden Elements (22; 122; 222) ausgespart ist, wobei der besagte Sitz auf eine Achse (X₂₄-X'₂₄; X₁₂₄-X'₁₂₄) zentriert ist, die zu der besagten vorderen Fläche im Wesentlichen senkrecht ist, wobei die besagte vordere Fläche zu der hinteren Fläche (26; 126; 226) der besagten Basis nicht parallel ist (β).

4. Komponente nach Anspruch 3, **dadurch gekennzeichnet, dass** die besagte vordere Fläche (25; 125; 225) und die besagte hintere Fläche (26; 126; 226) zusammen einen Winkel (β) im Bereich von 2° bis 18° bilden.

5. Komponente nach einem der Ansprüche 3 oder 4, **dadurch gekennzeichnet, dass** die besagte Basis (21; 121; 221) mit einer achsensymmetrischen Oberfläche (30; 130) versehen ist, die auf eine Achse (X₂₄-X'₂₄; X₁₂₄-X'₁₂₄) zentriert ist, die zu der vorderen Fläche (25; 125; 225) senkrecht ist, wobei die besagte Oberfläche dazu geeignet ist, mit einer internen Oberfläche (31) des besagten Elements (22; 122; 222) zusammenzuwirken, um das besagte Element an der besagten Basis zu zentrieren und zu immobilisieren.

6. Komponente nach einem der vorhergehenden Ansprüche, **dadurch gekennzeichnet, dass** sie mit einem Verankerungsschaft (127; 227) versehen ist, der sich in eine Richtung erstreckt, die wenigstens zu einem Teil der besagten hinteren Fläche (126; 226) nicht senkrecht ist.

7. Komponente nach einem der vorhergehenden Ansprüche, **dadurch gekennzeichnet, dass** das besagte, die konvexe Gelenkoberfläche (S₂) definierende Element (322; 422) mit einer Schürze (322b; 422b) versehen ist, die in Bezug auf die besagte Achse nicht symmetrisch ist (γ) und die besagte konvexe Gelenkoberfläche (S₂) verlängert und in der wenigstens teilweise ein Sitz (332) für die Aufnahme wenigstens eines Teils der besagten Basis (321; 421) definiert ist.

8. Komponente nach Anspruch 7, **dadurch gekennzeichnet, dass** die besagte Schürze (322b; 422b) im Wesentlichen die Form eines Torusteilstücks hat.

9. Komponente nach einem der Ansprüche 7 oder 8, **dadurch gekennzeichnet, dass** das besagte Element (322; 422) mit einer Durchbohrung (334) für den Durchgang (F₃) einer Betätigungsvorrichtung für Betätigungsmittel zum Befestigen des besagten Elements an der besagten Basis (321; 421) versehen ist, wobei sich die Durchbohrung im Wesentlichen in einer Richtung (X₃₃₄-X'₃₃₄) erstreckt, die zu der besagten Oberfläche (S₂) global senkrecht ist und nicht mit der besagten Achse (X₂-X'₂) zusammenfällt (γ).

10. Satz von Gelenkpfannenkomponenten nach Anspruch 1 für Schulterprothese, in dem jede Komponente eine Basis (21; 121; 221; 321; 421) aufweist, die an der Gelenkpfanne einer Schulter immobilisierbar ist und ein Element (22; 122; 222; 322; 422) aufweist, das dazu vorgesehen ist, an der Basis montiert zu werden, und eine konvexe Oberfläche bildet, die auf eine Symmetrieachse (X₂-X'₂) zentriert ist, **dadurch gekennzeichnet, dass** die Orientierung (β, γ) der besagten Symmetrieachse (X₂-X'₂) in Bezug auf die hintere Fläche (26; 126; 226; 326; 426) jeder Komponente, die dazu bestimmt ist, sich an der Gelenkpfanne (G) abzustützen, von einer Komponente zur Nächsten (2a-2d; 302, 402) variabel ist.

11. Satz von Komponenten nach Anspruch 10, **dadurch gekennzeichnet, dass** die Komponenten gemäß Anspruch 3 ausgebildet sind und dass der Winkel (β) zwischen der vorderen Fläche (25; 125; 225) und der hinteren Fläche (26; 126; 226) der Basen (21; 121; 221) von einer Komponente zur Nächsten (2a-2d) unterschiedlich ist.

12. Satz von Komponenten nach Anspruch 10, **dadurch gekennzeichnet, dass** die Komponenten gemäß Anspruch 7 ausgebildet sind und dass die Abmessungen (γ) der Schürzen (322b, 422b) von einer Komponente zur Nächsten (302, 402) unterschiedlich sind.

13. Schultervollprothese, **dadurch gekennzeichnet, dass** sie eine Gelenkpfannenkomponente (2; 102; 202; 302; 402) nach einem der Ansprüche 1 bis 9 aufweisen.
